# EUROPEAN PATENT APPLICATION

(11) **EP 4 814 490 A1**
(43) Date of publication of application: **30.09.2026**
(21) Application number: 24894354.0
(22) Date of filing: 26.09.2024
(51) Int. Cl.: G01N 33/569, G01N 33/543, G16B 40/20, G16B 45/00

(54) **DIAGNOSTIC SYSTEM FOR DISTINGUISHING BACTERIAL INFECTION AND VIRAL INFECTION USING MYXOVIRUS RESISTANCE PROTEIN A AND C-REACTIVE PROTEIN**

(30) Priority: 21.11.2023 KR 20230162048
(71) Applicant: Boditech Med Inc., Chuncheon-si, Gangwon-do 24398 (KR)
(72) Inventor: EVANGELOS, J. Giamarellos-Bourboulis, 12482 Athenes (GR); JEONG, Jeonghyeok, Chuncheon-si, Gangwon-do 24363 (KR); SHIM, Yumi, Chuncheon-si, Gangwon-do 24221 (KR); LEE, Yoonsuk, Chuncheon-si, Gangwon-do 24414 (KR)
(74) Representative: Plasseraud IP
(86) International application number: PCT/KR2024/014556
(87) International publication number: WO 2025/110458

(57) **Abstract**

A diagnostic system for distinguishing between bacterial infection and viral infection using Myxovirus resistance proteins A and C-reactive proteins, which is characterized in that a detector antibody binds to an antigen in a blood sample to form an antigen-antibody complex, and the antigen-antibody complex, while moving to a nitrocellulose matrix, is captured by other antibodies immobilized on a test strip, allowing the quantitative detection of MxA and CRP levels in the blood sample.

## Description

### TECHNICAL FIELD

The present disclosure relates to a diagnostic system capable of safely distinguishing between viral and bacterial infections by using a combination of myxovirus resistance protein A (hereinafter referred to as "MxA") and C-reactive protein (hereinafter referred to as "CRP").

More specifically, the present disclosure enables quantitative measurement of biomarkers MxA and CRP and derivation of the bacterial infection probability using an equation containing MxA and CRP values.

### BACKGROUND

Viral and bacterial infections are major causes of disease, mortality, and healthcare cost. Approximately 80% of all antibiotics are prescribed in primary care settings, with the majority used for respiratory infections.

However, most acute respiratory infections are viral in origin, and clinicians need tools to reinforce their clinical judgment in order to avoid unnecessary antibiotic prescriptions when in doubt.

Such tools must be point-of-care (POC) and easily provide the likelihood of viral or bacterial infection.

So far, procalcitonin has been a biomarker that can be selected for use to reduce unnecessary antibiotic consumption. Procalcitonin is produced in cells and organs in response to proinflammatory cytokines caused by bacterial infections and released in the blood.

This has been demonstrated through randomized clinical trials. However, two or more biomarkers often need to be used together for accurate patient evaluation.

Preferably, at least one biomarker needs to indicate the likelihood of viral infection and the other the likelihood of bacterial infection. Such necessity has been demonstrated by several microbiological studies showing that lower respiratory tract infections frequently involve mixed viral and bacterial etiologies.

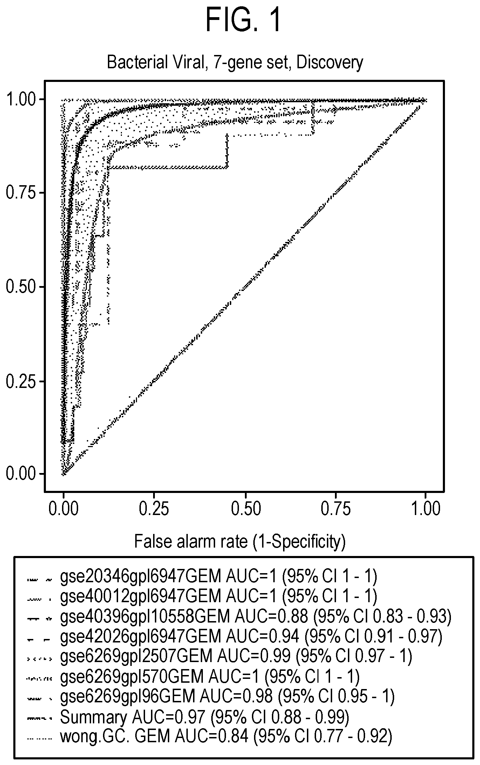

Korean Patent No. 10-2515555 (March 28, 2023), a method for diagnosing bacterial and viral Infections, relates to the use of a biomarker capable of determining whether a patient with acute inflammation has a bacterial or viral infection (See FIG. 1).

This patent involves a method for providing information useful to distinguish a patient diagnosed with an infection as having either a viral or bacterial infection, the method comprising:
a) measuring the expression levels of two biomarkers within a biological sample from the patient, wherein the biomarkers are transcripts selected from CTSB, ISG15, OASL, IFI27, and JUP; and
b) analyzing the respective expression levels of the biomarkers along with respective reference value ranges of the biomarkers to calculate the metascore that distinguishes the patient as having a viral or bacterial infection.

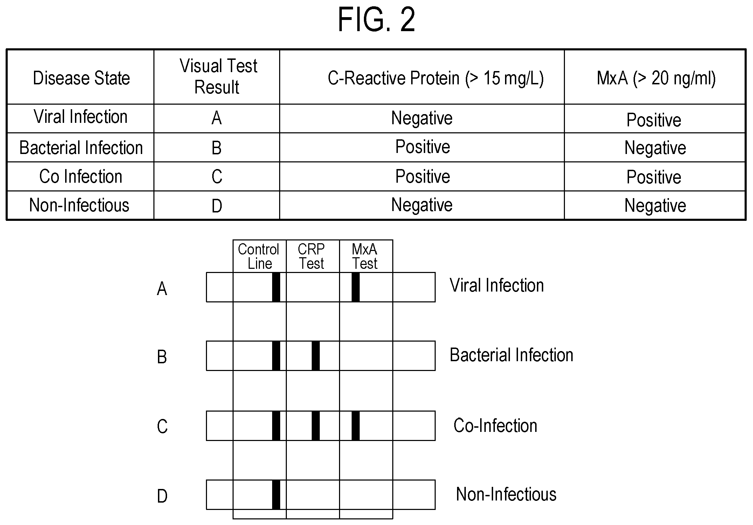

U.S. Patent No. 9,910,036 B2 (March 6, 2018) relates to a complex detection device and method for viral and bacterial infections (see FIG. 2).

This patent is directed to a lateral flow assay device, which detects and distinguishes between viral and bacterial infections. The complex diagnostic device is designed to test viral and bacterial infection markers on the spot and help distinguish between viral and bacterial infections quickly and effectively.

In a preferred embodiment, the bacterial marker is CRP. In another preferred embodiment, the viral marker is MxA. In some embodiments, it is not necessary to lyse cells in samples prior to applying the markers to the device.

### [Prior Art Document]

(Patent Document 1) KR 10-2515555 B1 (March 28, 2023)
(Patent Document 2) US 9,910,036 B2 (March 6, 2018)

### DISCLOSURE

### TECHNICAL PROBLEM

Examples of candidate biomarkers include myxovirus resistance protein (MxA) and C-reactive protein (CRP).

Myxovirus resistance protein (MxA) is a large interferon-inducible GTPase (GTP hydrolase) involved in the control of intracellular pathogens. In humans, two Mx homologs mediate antiviral activity against a broad range of viruses including SARS-CoV-2. Elevated MxA indicates increased endogenous interferon production mediated by viral activation, and may be used as a marker of viral infection.

C-reactive protein (CRP) is a well-known cytokine-induced acute phase protein. Its blood level rises in response to non-specific infections caused by various possible factors, rather than a specific infections.

The measurement of the two proteins may be integrated into existing commercially available test devices, allowing for qualitative approaches to detect elevated levels of MxA, CRP, or both.

However, the existing approaches have limitations in personalized diagnosis. The qualitative approaches do not account for individual patient circumstances, and moreover, many patients may be co-infected with viruses and bacteria, and therefore, more accurate measurements are needed for correct decision making.

### TECHNICAL SOLUTION

The present disclosure has been made to solve the above-mentioned issues, and an aspect of the present disclosure is to provide a completely different approach to analysis of MxA and CRP as diagnostic tools for viral and bacterial infections.

Continuous measurements of the two proteins are analyzed using a discovery cohort and a validation cohort. The discovery cohort consists of patients with definitive infection and the validation cohort consists of patients with a high probability of viral, bacterial, or co-infection etiology. The cutoffs developed in the discovery cohort are validated in the validation cohort.

### ADVANTAGEOUS EFFECTS

The algorithm used in the diagnostic system of the present disclosure provides the usability of MxA and CRP as tools for distinguishing between viral acute respiratory disease and bacterial acute respiratory disease in the emergency room, reporting a sensitivity and a specificity of 93.2% and 88.4% for bacterial infection, respectively.

The use of the algorithm of the present disclosure can significantly reduce antibiotic administration to viral-infected patients from 18.4% to 11.6%.

The algorithm of the present disclosure presents a novel approach to the interpretation of MxA and CRP for the differential diagnosis between viral and bacterial infections.

Both the biomarkers MxA and CRP are quantitatively measured and then used to calculate the probability of bacterial infection, and the results are interpreted in view of the MxA to CRP ratio.

According to the algorithm of the present disclosure, a combination of MxA and CRP make it possible to safely distinguish between viral and bacterial infections.

The algorithm of the present disclosure can determine the infection probability by an equation containing MxA and CRP values.

According to the approach of the present disclosure, the overall sensitivity and overall NPV for bacterial infection are greater than 90%.

### DESCRIPTION OF DRAWINGS

FIG. 1 shows an example of a Korean Patent involved in an existing diagnosis of viral and bacterial infections.
FIG. 2 shows an example of a U.S. Patent involved in an existing diagnosis of viral and bacterial infections.
FIG. 3 shows a classification diagram of patients according to the study of the present disclosure.
FIG. 4 shows result tables obtained by analyzing MxA and CRP measurement values and actual patients according to the study of the present disclosure.
FIG. 5 shows the analysis results of blood MxA and CRP levels in viral infections (136 patients for group b) and bacterial infections (131 patients for group c) according to the study of the present disclosure.
FIG. 6 shows the logistic regression analysis results according to the study of the present disclosure.
FIG. 7 shows a receiver operating characteristics (ROC) curve of the probability (p) of bacterial infection diagnosis.
FIG. 8 shows a ROC curve of the MxA/CRP ratio for the diagnosis of viral infection.
FIG. 9 shows a table obtained by integrating the bacterial infection probability (p) and the MxA/CRP ratio into a single diagnostic rule.
FIG. 10 shows MxA and CRP values for the diagnosis of bacterial and viral infections in the validation cohort.
FIG. 11 shows a table obtained by integrating the bacterial infection probability (p) and the MxA/CRP ratio into a single diagnostic rule in the validation cohort.
FIG. 12 shows the diagnosis values of MxA and CRP in patients with co-infection.
FIG. 13 shows the MxA/CRP ratio for the diagnosis of unfavorable outcome.

### BEST MODE FOR DISCLOSURE

The terminology used herein is only for the purpose of describing particular embodiments and is not intended to limit the disclosure. Singular forms include plural forms, unless otherwise indicated by the context. It is to be understood that the terms such as "comprise", "contain", "include" or "have" are used herein to designate the presence of characteristic, number, step, operation, element, component, or a combination thereof, and not to preclude the presence or the possibility of adding one or more of other characteristics, numbers, steps, operations, elements, components or combinations thereof.

Unless otherwise defined, all terms including technical and scientific terms used herein have the same meaning as commonly understood by a person skilled in the art to which this disclosure pertains. Terms defined in commonly used dictionaries shall be interpreted as having meanings consistent with those understood in the context of the relevant art, and, unless expressly defined in the present application, shall not be interpreted in an idealized or overly formal sense.

### MODE FOR INVENTION

### [Study Design]

The present disclosure is based on a study conducted in the Department of internal medicine located at public hospitals in Greece from July 2022 to February 2023.

The study is a sub-study of the clinical trials ACCESS and ImmunoSep, which were approved by the Greek National Ethics Committee (1912 122/20 and 2/21) and the Greek National Agency for Medicines (1912 IS113/20 and IS008/21). Written informed consent was provided by patients or their legal representatives, and patients who were assessed for eligibility in both trials were involved in the study.

The enrolled patients needed to meet all inclusion criteria: a) male or female adults aged 18 years or older; and b) clinical signs of infection, such as fever, cough, dyspnea, sore throat, headache, nasal congestion, or diarrhea.

Exclusion criteria included age under 18 years, chronic oral or intravenous use of corticosteroids, known infections caused by human immunodeficiency virus, neutropenia, chronic anticytokine therapy, pregnancy, or lactation.

Forty patients meeting no exclusion criteria and having no signs of active infection were enrolled as a control group.

### [Testing and Data Collection]

All the study participants had the following physical examination, including current and past medical history:

complete blood count (CBC), biochemistry, blood gases, and procalcitonin; chest x-ray, and high-resolution chest computed tomography if indicated; nasopharyngeal swab for SARS-CoV-2 PCR testing, and upper and lower respiratory tract testing using BioFire's FilmArray panels if indicated; urine testing for Streptococcus pneumoniae and Legionella antigens using BinaxNow assay, and stool toxin assay for Clostridioides difficile; and blood, sputum, or urine culture.

The sequential organ failure assessment (SOFA) score and Charlson's comorbidity index (CCI) were calculated. The patients were followed up for 28 days for survival.

In parallel with routine blood sampling, 4 mL of venous whole blood was collected into one EDTA-coated tube (Vacutainer, Becton Dickinson, Cockeysville Md) and analyzed using MxA/CRP testing.

### [MxA/CRP Testing]

MxA/CRP testing employs a fluorescent lateral flow immunoassay system for the quantitative determination of MxA and CRP in the human blood.

In the immunoassay system, detector antibodies bind to antigens in a blood sample to form antigen-antibody complexes, and the antigen-antibody complexes, while moving along a nitrocellulose matrix, are captured by other antibodies immobilized on a test strip.

Larger amounts of antigens contained in the blood sample form increased amounts of antigen-antibody complexes, resulting in stronger fluorescent signals generated by the detection antibodies, leading to the quantitative detection of blood MxA and CRP levels within the blood sample.

The immunoassay system includes a plurality of cartridge packages, each including three components, that is, a cartridge, a detector, and a dilution part.

The cartridge is composed of a membrane called a test strip, with anti-MxA and anti-CRP antibodies on test lines, CRP Ag on an antigen line, and chicken IgY on a control line.

The detector includes fine particles containing an anti-MxA-fluorescence complex, an anti-CRP-fluorescence conjugate pair, an anti-chicken IgY-fluorescence conjugate pair, and sodium azide as a preservative in phosphate buffered saline (PBS).

The dilution part also contains sodium azide as a preservative in phosphate buffered saline (PBS).

Preferably, the immunoassay system may quantitatively measure MxA and CRP within the blood sample and display the blood MxA and CRP levels as values in units of ng/mL and mg/L, respectively.

The detection ranges of the immunoassay system are 10.0-300.0 ng/mL for MxA and 1.0-200.0 mg/L for CRP.

### [Clinical Assessment]

The infection status of the patients may be determined as follows on the basis of the result values of blood MxA and CRP levels according to the present disclosure.$MxA \geq 15.00 ng / mL$
① CRP < 10.00 mg/L: Viral infection
② 10.00 < CRP < 20.00 mg/L: Viral infection, and low likelihood of bacterial infection
③ 20.00 < CRP < 100.00 mg/L: Likely viral and bacterial co-infection
④ CRP ≥ 100.00 mg/L: High likelihood of viral and bacterial co-infection$MxA < 15.00 ng / mL$
   ① 10.00 < CRP < 20.00 mg/L: Possible bacterial infection
   ② 20.00 < CRP < 100.00 mg/L: Likely bacterial infection
   ③ CRP ≥ 100.00 mg/L: High likelihood of bacterial infection
   ④ CRP < 10.00 mg/L: Non-infection

According to the assessment criteria, the patients may be classified into the seven categories below:
a) Non-infection: Patients with no infection.
b) Viral infection alone: Infection is limited to a viral origin, with no bacterial pathogens detected.
c) Bacterial infection alone: Infection is limited to a bacterial origin, with no viral pathogens detected.
d) Viral/bacterial co-infected: Infection is caused by both viral and bacterial origins.
e) High likelihood of viral infection: No viral pathogens were detected, but there is a high likelihood of viral infection.
f) High likelihood of bacterial infection: No bacterial pathogens were detected, but there is a high likelihood of bacterial infection.
g) Viral infection alone with high likelihood of bacterial co-infection: Infection is limited to a viral origin, with no bacterial pathogens detected but a high likelihood of bacterial co-infection.

A main purpose of the present disclosure is to develop an algorithm that accurately classifies a patient as having viral infection, bacterial infection, or co-infection by using the blood levels of the two biomarkers MxA and CRP and ratio thereof.

Another purpose of the present disclosure is to provide early prognosis of negative results after 28 days from onset by developing cutoffs of MxA, CRP, and ratio thereof.

### <Statistical Analysis>

Qualitative variables are expressed as frequencies with 95% confidence intervals (CIs), and medians with 95% confidence intervals (CIs). Comparisons of qualitative variables between groups are conducted by Fisher's test. Comparisons of quantitative variables between groups are conducted by the Mann-Whitney U test after Bonferroni correction for multiple testing.

The discovery cohort consists of patients with viral and bacterial co-infection.

For the patients, two novel approaches are followed, that is, the infection probability (p) and the MxA/CRP ratio.
a) The diagnostic cutoff values of biomarkers MxA and CRP are combined to determine diagnostic performance.
   According to an embodiment of the present disclosure, the diagnostic cutoff values are MxA (>15 ng/mL) and CRP (>10 mg/L), respectively.
b) The logistic regression analysis is performed to create an equation capable of defining a bacterial infection probability (p).
c) The receiver operator characteristic (ROC) curve analysis is performed by using the Youden index to define a probability cutoff with an optimal trade-off;
d) The area under the curve (AUC) and 95% Cls are calculated;
e) The MxA to CRP ratio is calculated to plot an ROC curve providing the optimal trade-off for viral infection diagnosis.
f) Two cutoffs for the bacterial infection probability (p) and the MxA to CRP ratio are integrated into a definitive diagnostic rule for distinguishing between bacterial and viral infections.

The definitive diagnostic rule is validated in the validation cohort

In addition, the cutoffs are applied to patients with viral/bacterial co-infection and patients with viral infection and a high likelihood of bacterial co-infection.

The sensitivity, specificity, positive predictive value (PPV), and negative predictive value (NPV) are calculated.

A p-value of < 0.05 is considered statistically significant.

### [Study population]

A total of 537 patients were enrolled, and 40 uninfected patients (group a) were included in the control group.

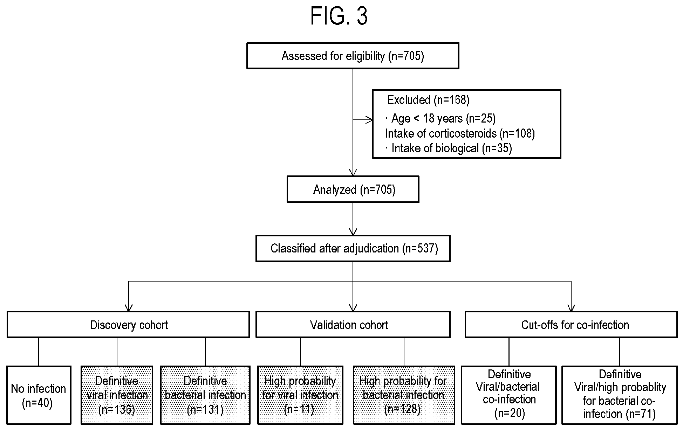

The discovery cohort included 267 patients. Of these, 136 patients showed definitive viral infection, and 131 patients showed definitive bacterial infection (see FIG. 3).

The other patients were studied in the validation cohort

Overall, the average age of the patients was 68.1 years, and male accounted for 60.7%.

The average SOFA was 3.27 and the average CCI was 3.9.

Overall, the mortality rate after 28 days was 19.0%.

### [Diagnostic performance using cutoffs of biomarkers MxA and CRP]

In the present disclosure, the diagnostic analysis of MxA and CRP is performed by a cartridge consisting of a test strip with anti-MxA and anti-CRP antibodies on test lines, CRP Ag on an antigen line, and chicken IgY on a test line.

The diagnostic performance for MxA and CRP is analyzed and determined from all the enrolled patients by using cutoff values of a diagnostic kit analysis system.

In the present disclosure, the diagnostic performance for diagnostic cutoff values of the two biomarkers, MxA (>15 ng/mL) and CRP (>10 mg/L), is as follows.

Among 189 patients with a blood MxA level equal to or above the cutoff value (≥15 ng/mL), 181 patients had definitive viral infection alone, with a PPV of 95.8%.

Whereas, among 78 patients with a blood MxA level equal to or below the cutoff value (<15 ng/mL), 32 patients had no infection, with a NPV of 41.0%.

In addition, among 227 truly positive patients, 181 patients were determined to be positive by the test, with 79.7% sensitivity.

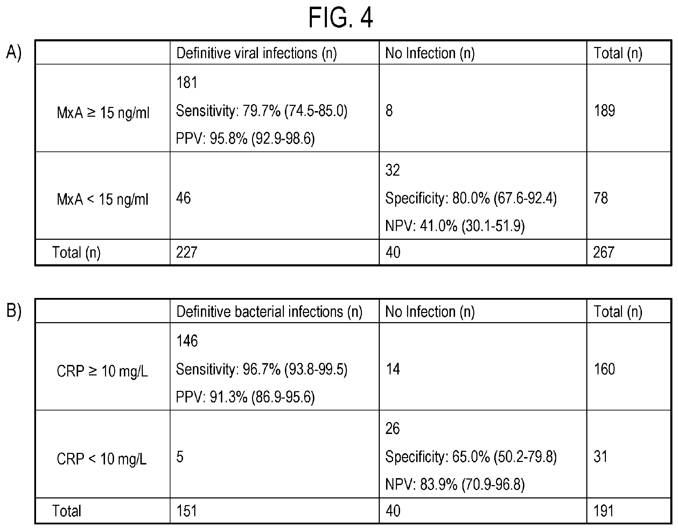

Whereas, among 40 truly negative patients, 32 patients were determined to be negative by the test, with 80.0% specificity (see FIG. 4A).

Among 160 patients with a blood CRP level equal to or above the cutoff value (≥10 mg/L), 146 patients had definitive bacterial infection alone, with a PPV of 91.3%.

Whereas, among 31 patients with a blood MxA level equal to or below the cutoff value (<10 mg/L), 26 patients had no infection, with a NPV of 83.9%.

In addition, among 151 truly positive patients, 146 patients were determined to be positive by the test, with a sensitivity of 96.7%.

Whereas, among 40 truly negative patients, 26 patients were determined to be negative by the test, with a specificity of 65.0%. (see FIG. 4B)

### [Analysis of primary assessment variables]

Finally, a comparison was conducted on 267 patients from the discovery cohort, defined as having viral infection (136 patients for group b) and bacterial infection (131 patients for group c).

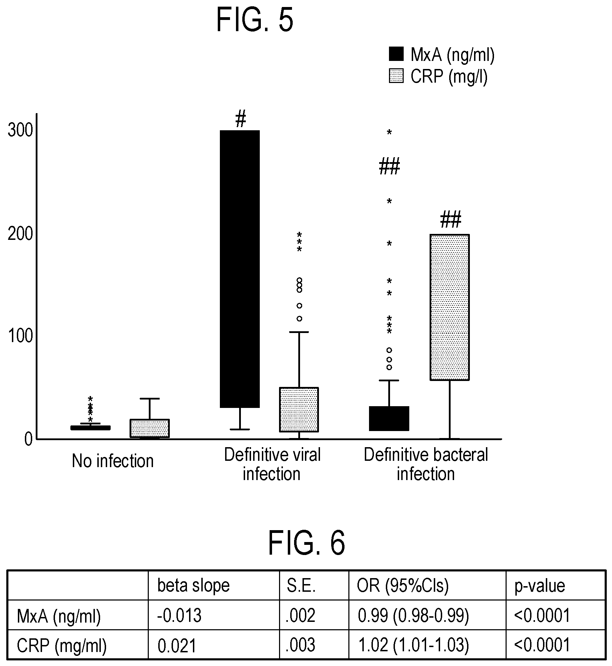

The results showed that the MxA level was very high in the viral infection and the CRP level was high in bacterial infection (FIG. 5).

After the logistic regression analysis, the equation below for deriving the bacterial infection probability (p) is created by taking the absolute values of MxA and CRP.

FIG. 6 shows the logistic regression analysis results regarding the effect of MxA and CRP on the diagnosis of bacterial infection.

It can be seen from the analysis results that an increase in CRP indicates a bacterial infection and an increase in MxA indicates a viral infection.

The following equation is created from the analysis results.$ln \left(\frac{p}{1 - p}\right) = - 0.414 - 0.013 MxA + 0.021 CRP$

P is the bacterial infection probability, and Equation 1 is transformed to derive the following equation.$p = \frac{1}{1 + {e}^{- \left(-0.404-0.013MxA+0.021CRP\right)}}$

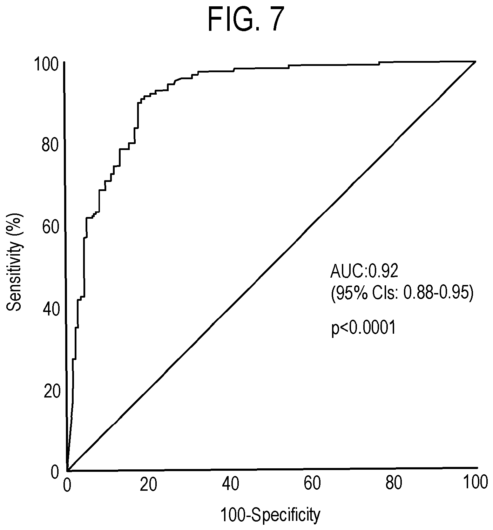

FIG. 7 shows a receiver operating characteristics (ROC) curve of the probability (p) for bacterial infection diagnosis.

The analysis was conducted on 267 patients with viral infection alone (n=136) and bacterial infection alone (n=131).

The area under the curve (AUC) of the ROC curve was 0.92, with a 95% confidence interval (CI) of 0.88-0.95, so as to establish bacterial infection diagnosis by clearly discriminating between bacterial and viral infections using a bacterial infection probability (p) ≥ 0.5.

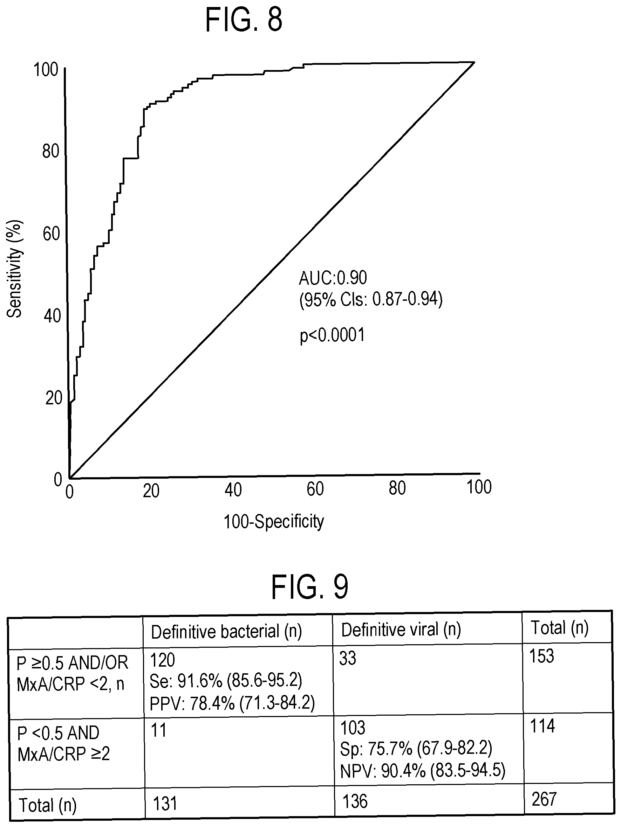

FIG. 8 shows a ROC curve of the MxA/CRP ratio for viral infection diagnosis.

The analysis was conducted on 267 patients with viral infection alone (n=136) and bacterial infection alone (n=131).

The area under the curve (AUC) of the ROC curve was 0.90, with a 95% confidence interval (CI) of 0.87-0.94, so as to establish diagnosis by clearly discriminating between bacterial and viral infections using a MxA/CRP ratio of < 2.

Then, the bacterial infection probability (p) and the MxA/CRP ratio are integrated into a single diagnostic rule (see FIG. 9).

Among 153 patients with a bacterial infection probability (p) of ≥ 0.5 and/or a MxA/CRP of < 2, 120 patients had definitive bacterial infection, with a PPV of 78.4%.

Whereas, among 114 patients with a bacterial infection probability (p) of < 0.5 and/or a MxA/CRP ratio of ≥ 2, 103 patients had definitive viral infection, with a NPV of 90.4%.

Additionally, among the 131 patients with definitive bacterial infection, 120 patients were determined to have an infection probability (p) of ≥ 0.5 and/or MxA/CRP < 2 in the test, with a sensitivity of 91.6%.

Whereas, among the 136 patients with definitive viral infection, 103 patients had an infection probability (p) of < 0.5 and/or MxA/CRP ≥ 2, with a specificity of 75.7%.

In the diagnostic rule, an NPV of 90% or higher corresponds to a very important consequence not to miss patients in need of antibiotics.

In the validation cohort, MxA was higher in patients with a higher probability of viral infection, and CRP was higher in patients with a higher probability of bacterial infection.

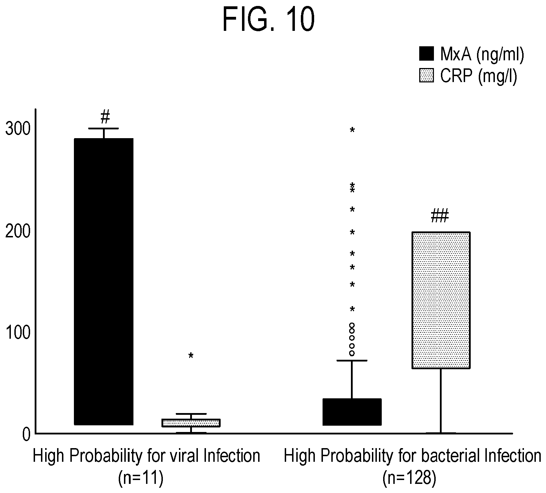

FIG. 10 shows MxA and CRP for the diagnosis of bacterial and viral infections in the validation cohort.

The figure illustrates the blood MxA and CRP levels in patients with a high probability of viral infection (n=11) and patients with a high probability of bacterial infection (n=128).

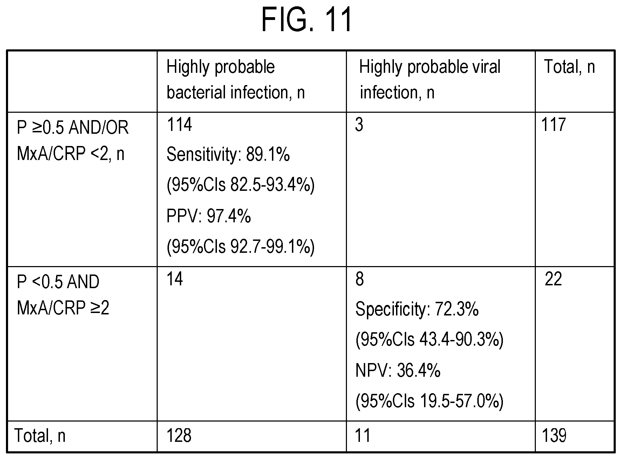

The application of the cutoffs developed in the discovery cohort resulted in a sensitivity of 89.1% for bacterial infection and a PPV of 97.4% for bacterial infection. However, the NPV of bacterial infection was 36.4%, which was lower than that in the discovery cohort (see FIG. 11).

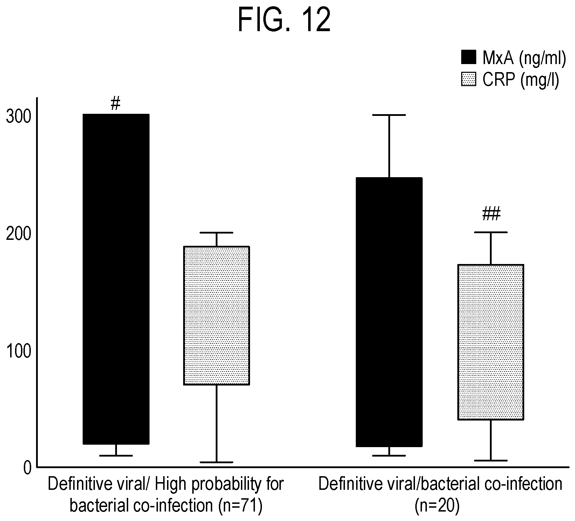

Both MxA and CRP were high in patients with co-infection (FIG. 12).

Among 71 patients classified as having clear viral infection and a high probability of bacterial co-infection (Group g), 46 patients (64.8%) were determined to be positive by an integrated diagnostic system using the probability (p) and the MxA/CRP.

This corresponds to 14 out of 20 patients (70.0%) with definitive viral/bacterial co-infection.

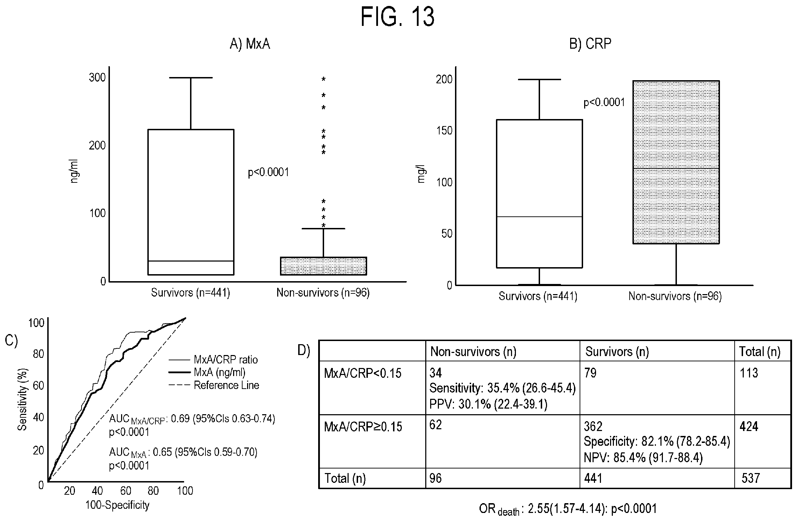

FIG. 13 shows the MxA/CRP ratio for negative diagnosis results.
Panel A) shows a comparison of MxA between 28-day survivors and non-survivors (p-value for comparison provided). The MxA levels were lower in the non-survivors than in the survivors (panel A); and
Panel B) shows a comparison of CRP between 28-day survivors and non-survivors (p-value for comparison provided). The CRP levels were higher in the non-survivors (panel B).
Panel C) shows an ROC curve of MxA and MxA/CRP ratio for predicting unfavorable outcomes.

The MxA/CRP ratio showed a higher AUC on the ROC curve for predicting negative outcomes after 28 days (Panel C).

Panel D) shows prognostic performance of an MxA/CRP ratio of less than 0.15 for non-survivors after 28 days. Analysis involved 537 patients, 95% confidence intervals for non-survivors with an MxA/CRP ratio of less than 0.15 are provided (odds ratio = 2.55).

Values of the MxA/CRP ratio lower than 0.15 were associated with an NPV of 85.4% for exclusion of risk for death (Panel D).

### INDUSTRIAL APPLICABILITY

The present disclosure can be used to accurately classify patients as having bacterial infection, viral infection, or co-infection by using the blood levels of the two biomarkers MxA and CRP and the ratio thereof.

## Claims

1. A diagnostic system for distinguishing between bacterial and viral infections by using myxovirus resistance protein A and C-reactive protein, in which detector antibodies bind to antigens in a blood sample to form antigen-antibody complexes, and the antigen-antibody complexes, while moving along a nitrocellulose matrix, are captured by other antibodies immobilized on a test strip, thereby quantitatively detecting blood MxA and CRP levels in the blood sample.

2. The diagnostic system of claim 1, wherein the immunoassay system comprises a plurality of cartridge packages, each comprising a cartridge, a detector, and a dilution part.

3. The diagnostic system of claim 2, wherein the cartridge is composed of a membrane called a test strip, which has anti-MxA and anti-CRP antibodies on test lines, CRP Ag on an antigen line, and chicken IgY on a control line.

4. The diagnostic system of claim 2, wherein the detector comprises fine particles containing an anti-MxA-fluorescent complex, an anti-CRP-fluorescent conjugate pair, an anti-chicken IgY-fluorescent conjugate pair, and sodium azide as a preservative in phosphate buffered saline (PBS).

5. The diagnostic system of claim 2, wherein the dilution part contains sodium azide as a preservative in phosphate buffered saline (PBS).

6. The diagnostic system of claim 1, wherein the blood MxA and CRP levels within the blood sample are displayed as values in units of ng/mL and mg/L, respectively.

7. The diagnostic system of claim 1, wherein the detection ranges of MxA and CRP are 10.0-300.0 ng/mL and 1.0-200.0 mg/L, respectively.

8. The diagnostic system of claim 1, wherein the biomarkers MxA and CRP have diagnostic cutoff values of 15 ng/mL and 10 mg/L, respectively.

9. The diagnostic system of claim 1, wherein the absolute values of the biomarkers MxA and CRP are taken to derive a bacterial infection probability (p) through Equation 1 below. $ln \left(\frac{p}{1 - p}\right) = - 0.414 - 0.013 MxA + 0.021 CRP$

10. The diagnostic system of claim 1, wherein the absolute values of the biomarkers MxA and CRP are taken to derive a bacterial infection probability (p) through Equation 2 below. $p = \frac{1}{1 + {e}^{- \left(-0.404-0.013MxA+0.021CRP\right)}}$

11. A diagnostic method for distinguishing between bacterial and viral infections by using myxovirus resistance protein A and C-reactive protein, the method comprising:
a) combining diagnostic cutoff values of biomarkers MxA and CRP to determine diagnostic performance;
b) performing logistic regression analysis to create an equation capable of defining a bacterial infection probability (p);
c) performing receiver operator characteristic (ROC) curve analysis by using the Youden index to define a probability cutoff with an optimal trade-off;
d) calculating the area under the curve (AUC) and 95% Cls;
e) calculating the MxA to CRP ratio to plot an ROC curve providing the optimal trade-off for viral infection diagnosis; and
f) integrating cutoffs for the bacterial infection probability (p) and the MxA to CRP ratio into a definitive diagnostic rule for distinguishing between bacterial and viral infections.

12. The method of claim 11, wherein in step (a), the diagnostic cutoff values of the biomarkers MxA and CRP are 15 ng/mL and 10 mg/L, respectively.

13. The method of claim 11, wherein the equation created in step (b) is as shown in Equation 1 below. $ln \left(\frac{p}{1 - p}\right) = - 0.414 - 0.013 MxA + 0.021 CRP$

14. The method of claim 11, wherein the equation created in step (b) is as shown in Equation 2 below. $p = \frac{1}{1 + {e}^{- \left(-0.404-0.013MxA+0.021CRP\right)}}$

15. The method of claim 11, wherein in step (f), the cutoff values for the bacterial
infection probability (p) and the MxA to CRP ratio are 0.5 and 2.0, respectively
